(19) Europäisches Patentamt
European Patent Office
Office européen des brevets

(11) EP 4 176 873 A1

(12) **EUROPEAN PATENT APPLICATION**
published in accordance with Art. 153(4) EPC

(43) Date of publication:
**10.05.2023 Bulletin 2023/19**

(21) Application number: **21833986.9**

(22) Date of filing: **02.07.2021**

(51) International Patent Classification (IPC):
***A61K 31/122*** (2006.01) ***A61P 13/02*** (2006.01)
***A61P 13/10*** (2006.01)

(52) Cooperative Patent Classification (CPC):
**A61K 31/122; A61P 13/02; A61P 13/10**

(86) International application number:
**PCT/JP2021/025056**

(87) International publication number:
**WO 2022/004867 (06.01.2022 Gazette 2022/01)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **03.07.2020 JP 2020115743**
**03.06.2021 JP 2021093454**

(71) Applicant: **Taiho Pharmaceutical Co., Ltd.**
**Chiyoda-ku, Tokyo 101-8444 (JP)**

(72) Inventor: **NAGAOKA, Makoto**
**Tokyo 101-0047 (JP)**

(74) Representative: **Hoffmann Eitle**
**Patent- und Rechtsanwälte PartmbB**
**Arabellastraße 30**
**81925 München (DE)**

## (54) URINARY SYMPTOM THERAPEUTIC AGENT

(57) The present invention addresses the problem of providing a voiding symptom therapeutic agent for lower urinary tract disorders accompanied by residual urine. The present invention provides a voiding symptom therapeutic agent for lower urinary tract disorders with a residual urine volume of 50 mL or more, the therapeutic agent containing 3-(15-hydroxypentadecyl)-2,4,4-trimethyl-2-cyclohexen-1-one or a salt thereof as an active ingredient.

EP 4 176 873 A1

## Description

Technical Field

Cross-Reference to Related Applications

This application claims priority to Japan Patent Application No. 2020-115743 filed on July 3, 2020 and Japan Patent Application No. 2021-093454 filed on June 3, 2021, the disclosures of which are incorporated herein by reference in their entirety. The present invention relates to a therapeutic agent for treating a lower urinary tract disorder accompanied by residual urine, the therapeutic agent comprising 3-(15-hydroxypentadecyl)-2,4,4-trimethyl-2-cyclohexen-1-one or a salt thereof as an active ingredient.

Background Art

Major pathologies of urinary storage disorders include overactive bladder (OAB). Partial denervation is observed in bladder biopsy specimens from OAB patients (Non-Patent Document 1 and 2). It is speculated that with the progression of denervation, the bladder smooth muscle causes an overreaction to acetylcholine (denervation supersensitivity), and detrusor overactivity (DO) occurs. Symptoms of OAB include urinary storage symptoms, such as urinary urgency, frequent urination (daytime and nighttime), and incontinence, which are often accompanied by involuntary contractions of the bladder (detrusor overactivity) during the storage phase.

It is known that the elderly may have both voiding and storage symptoms. It has been reported that 40% of elderly males who have lower urinary tract symptoms (LUTS) with no observation of bladder outlet obstruction (BOO), such as prostatic hyperplasia, have LUTS symptoms accompanied by only detrusor overactivity; that 31% have LUTS symptoms accompanied by only impaired detrusor contractility; and that 11% have LUTS symptoms accompanied by both detrusor overactivity and impaired detrusor contractility (Non-patent Literature (NPL) 3). Results of an epidemiological study reveal that LUTS include various symptoms, and that voiding and storage symptoms coexist (Non-Patent Literature (NPL) 4).

Chronic ischemia of the bladder is known as one pathogenesis of OAB. Recently, several reports suggest the possibility that chronic ischemia causes DO and changes in the ischemic state from mild to severe progress detrusor underactivity (DU) (Non-Patent Literature (NPL) 5, 6, and 7).

The usefulness of 3-(15-hydroxypentadecyl)-2,4,4,4-trimethyl-2-cyclohexen-1-one has been reported. Specifically, Patent Literature (PTL) 1 discloses that 3-(15-hydroxypentadecyl)-2,4,4,4-trimethyl-2-cyclohexen-1-one promotes nerve growth and is useful as a medicament for preventing or treating brain diseases, such as dementia. Patent Literature (PLT) 2 discloses that 3-(15-hydroxypentadecyl)-2,4,4,4-trimethyl-2-cyclohexen-1-one is useful as a therapeutic agent for treating dysuria. Patent Literature (PLT) 3 discloses that 3-(15-hydroxypentadecyl)-2,4,4,4-trimethyl-2-cyclohexen-1-one is useful as a therapeutic agent for treating diseases based on vesicourethral dyssynergia. Patent Literature (PLT) 4 discloses that 3-(15-hydroxypentadecyl)-2,4,4,4-trimethyl-2-cyclohexen-1-one is useful as a therapeutic agent for treating detrusor hyperactivity with impaired contractility (DHIC).

In order to evaluate the effectiveness and safety of 3-(15-hydroxypentadecyl)-2,4,4-trimethyl-2-cyclohexen-1-one on lower urinary tract disorders, such as DU and OAB, clinical studies were performed for patients with lower urinary tract disorders.

In 2018, a working group of the International Continence Society (ICS) reported on the standardization of terminology for lower urinary tract disorders associated with DU. In the report, the definition of the term "underactive bladder" (UAB) is discussed. According to this report (Non-Patent Literature (NPL) 8, UAB is "a symptom syndrome suggesting detrusor underactivity (DU), characterized by a slow urinary stream, hesitancy, and abdominal straining to void, with or without a feeling of incomplete bladder emptying, sometimes with storage symptoms." Clear definitions of diagnostic criteria etc. for UAB are awaited.

Citation List

Patent Literature

PTL 1: WO1999/008987
PTL 2: WO2002/066024
PTL 3: WO2015/046377
PTL 4: WO2017/022787

Non-patent Literature

NPL 1: Br. J. Urol. 60, pp. 519-22 (1987)
NPL 2: Urology 50 Suppl. 6A, pp. 57-67 (1997)
NPL 3: J. Urol. 162, pp. 142-146 (1999)
NPL 4: Approach to Lower Urinary Tract Dysfunction, Tokyo, pp. 2-8 (2007)
NPL 5: Neurourol. Urodyn., 33, pp. 54-58 (2014)
NPL 6: LUTS, 6, pp. 131-137 (2014)
NPL 7: Int Urol. Nephrol. 46 (Suppl 1), pp. 23-S27 (2014)
NPL 8: Neurourol. Urodyn., 37(8), pp. 2928-2931 (2018)

Summary of Invention

Technical Problem

An object of the present invention is to provide a voiding symptom therapeutic agent for a lower urinary tract disorder accompanied by residual urine.

Solution to Problem

The present inventors conducted extensive research to achieve the above object. As a result, the inventors found that 3-(15-hydroxypentadecyl)-2,4,4-trimethyl-2-cyclohexen-1-one represented by the following formula (I) (also referred to below as "Compound 1") is useful as a therapeutic agent for treating a voiding symptom in a patient having a lower urinary tract disorder with a residual urine volume of 50 mL or more.

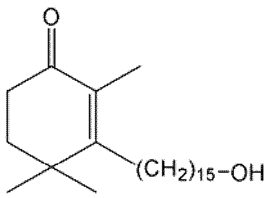
(I)

Specifically, the present invention includes the following [1] to [10].

[1] A therapeutic agent for treating a voiding symptom in a patient having a lower urinary tract disorder with a residual urine volume of 50 mL or more, the therapeutic agent comprising 3-(15-hydroxypentadecyl)-2,4,4-trimethyl-2-cyclohexen-1-one or a salt thereof.
[2] An agent for improving voiding efficiency in a patient having a lower urinary tract disorder with a residual urine volume of 50 mL or more, the agent comprising 3-(15-hydroxypentadecyl)-2,4,4-trimethyl-2-cyclohexen-1-one or a salt thereof.
[3] The therapeutic agent according to [1], wherein the patient has a lower urinary tract disorder with a residual urine volume of 100 mL or more.
[4] The agent for improving voiding efficiency according to [2], wherein the patient has a lower urinary tract disorder with a residual urine volume of 100 mL or more.
[5] A method using a residual urine volume as an indicator for a therapeutic effect of 3-(15-hydroxypentadecyl)-2,4,4-trimethyl-2-cyclohexen-1-one or a salt thereof on a lower urinary tract disorder, the method comprising determining a treatment using 3-(15-hydroxypentadecyl)-2,4,4-trimethyl-2-cyclohexen-1-one or a salt thereof to be effective when the residual urine volume is equal to or greater than coefficient T ($40 \leq T \leq 110$) mL.
[6] A therapeutic agent for treating a voiding symptom in a patient having a disease with reduced voiding efficiency with a residual urine volume of 50 mL or more, the therapeutic agent comprising 3-(15-hydroxypentadecyl)-2,4,4-trimethyl-2-cyclohexen-1-one or a salt thereof.
[7] An agent for improving voiding efficiency in a patient having a disease with reduced voiding efficiency with a residual urine volume of 50 mL or more, the therapeutic agent comprising 3-(15-hydroxypentadecyl)-2,4,4-trimethyl-2-cyclohexen-1-one or a salt thereof.
[8] The therapeutic agent according to [6], wherein the patient has a disease with reduced voiding efficiency with a

residual urine volume of 100 mL or more.

[9] The agent for improving voiding efficiency according to [7], wherein the patient has a disease with reduced voiding efficiency with a residual urine volume of 100 mL or more.

[10] A method using a residual urine volume as an indicator for a therapeutic effect of 3-(15-hydroxypentadecyl)-2,4,4-trimethyl-2-cyclohexen-1-one or a salt thereof on a disease with reduced voiding efficiency, the method comprising determining a treatment using 3-(15-hydroxypentadecyl)-2,4,4-trimethyl-2-cyclohexen-1-one or a salt thereof to be effective when the residual urine volume is equal to or greater than coefficient T ($40 \leq T \leq 110$) mL.

In other embodiments, the present invention is further directed to the following aspects.

- A pharmaceutical composition for treating a voiding symptom in a patient having a lower urinary tract disorder with a residual urine volume of 50 mL or more, the composition comprising 3-(15-hydroxypentadecyl)-2,4,4-trimethyl-2-cyclohexen-1-one or a salt thereof.

- A method for treating a voiding symptom in a patient having a lower urinary tract disorder with a residual urine volume of 50 mL or more, the method comprising administering an effective amount of 3-(15-hydroxypentadecyl)-2,4,4-trimethyl-2-cyclohexen-1-one or a salt thereof to the patient.

- A method for treating a voiding symptom in a patient having a lower urinary tract disorder, the method comprising the steps of

  measuring the residual urine volume in the patient;
  determining a treatment using 3-(15-hydroxypentadecyl)-2,4,4-trimethyl-2-cyclohexen-1-one or a salt thereof to be effective when the residual urine volume determined in the measurement step is equal to or greater than coefficient T ($40 \leq T \leq 110$) mL; and
  administering an effective amount of 3-(15-hydroxypentadecyl)-2,4,4-trimethyl-2-cyclohexen-1-one or a salt thereof to the patient for whom the treatment has been determined to be effective.

- Use of 3-(15-hydroxypentadecyl)-2,4,4-trimethyl-2-cyclohexen-1-one or a salt thereof for the production of a therapeutic agent for a voiding symptom in a patient having a lower urinary tract disorder with a residual urine volume of 50 mL or more.

- 3-(15-Hydroxypentadecyl)-2,4,4-trimethyl-2-cyclohexen-1-one or a salt thereof for use in the treatment of a patient having a lower urinary tract disorder with a residual urine volume of 50 mL or more.

Advantageous Effects of Invention

According to the present invention, a voiding symptom in a patient having a lower urinary tract disorder with a residual urine volume of 50 mL or more can be treated.

Description of Embodiments

Compound 1 is a known compound, and can be produced, for example, in accordance with the method described in WO1999/008987.

Examples of the "lower urinary tract disorder" in the present invention include detrusor underactivity, underactive bladder, overactive bladder, detrusor hyperactivity with impaired contractility, hypotonic bladder, atonic bladder, neurogenic bladder, detrusor-external urethral sphincter dyssynergia, frequent urination, nocturia, incontinence, benign prostatic hyperplasia, interstitial cystitis, chronic prostatitis, urolithiasis, and the like. The therapeutic agent of the present invention can also treat lower urinary tract disorders that are not caused by bladder-urethra dyssynergia. The therapeutic agent of the present invention can also treat lower urinary tract disorders other than diseases having both detrusor hyperactivity and impaired detrusor contractility. Further, the therapeutic agent of the present invention can treat lower urinary tract disorders other than detrusor hyperactivity with impaired contractility. In a preferred embodiment of the invention, the lower urinary tract disorder is, for example, at least one member selected from the group consisting of detrusor underactivity, underactive bladder, overactive bladder, detrusor hyperactivity with impaired contractility, hypotonic bladder, atonic bladder, and neurogenic bladder.

The patient having "a lower urinary tract disorder accompanied by residual urine" in the present invention is preferably a patient who has, as a lower urinary tract disorder, at least one member selected from the group consisting of detrusor underactivity, underactive bladder, overactive bladder, detrusor hyperactivity with impaired contractility,

hypotonic bladder, atonic bladder, and neurogenic bladder and who has a residual urine volume of 50 mL or more. More preferably, the patient having a lower urinary tract disorder accompanied by residual urine is a patient who has, as a lower urinary tract disorder, at least one member selected from the group consisting of detrusor underactivity, underactive bladder, overactive bladder, detrusor hyperactivity with impaired contractility, hypotonic bladder, atonic bladder, and neurogenic bladder and who has a residual urine volume of 100 mL or more. In the present invention, the residual urine volume refers to the amount of urine in the bladder after completion of voiding, and can be measured, for example, by transabdominal ultrasonography or urinary catheterization.

The "disease with reduced voiding efficiency" in the present invention refers to a disease in which a decrease in voiding efficiency is recognized from a decrease in voided urine volume and/or the maximum urinary flow rate as determined by uroflowmetry, or the presence of residual urine as determined by residual urine volume measurement. Examples of the disease with reduced voiding efficiency include detrusor underactivity, underactive bladder, detrusor hyperactivity with impaired contractility, hypotonic bladder, atonic bladder, neurogenic bladder, detrusor-external urethral sphincter dyssynergia, incontinence, benign prostatic hyperplasia, interstitial cystitis, chronic prostatitis, and urolithiasis. The "disease with reduced voiding efficiency" in the present invention also includes voiding symptoms in which the degree of decrease in voiding efficiency does not satisfy the diagnostic criteria for diseases with such voiding symptoms but that are ameliorated by improving voiding efficiency.

The "voiding symptoms" in the present invention refers to at least one member selected from the group consisting of reduction in voiding efficiency, decrease in voided volume, reduction in the maximal urinary flow rate, presence of residual urine, slow urinary stream, splitting ▪ spraying, intermittent stream, hesitancy, abdominal straining to void, terminal dribbling, feeling of residual urine, post-micturition dribbling, dysuria, and urinary retention. Preferably, the voiding symptom is a reduction in voiding efficiency.

Compound 1 may also be in the form of a pharmaceutically acceptable salt; specifically, an acid addition salt or a salt with a base. As long as the salt is a pharmaceutically acceptable salt, embodiments using such a salt are included within the scope of the present invention. Specific examples include acid addition salts with inorganic acids, such as hydrochloric acid, hydrobromic acid, hydroiodic acid, sulfuric acid, nitric acid, and phosphoric acid; acid addition salts with organic acids, such as formic acid, acetic acid, propionic acid, oxalic acid, malonic acid, succinic acid, fumaric acid, maleic acid, lactic acid, malic acid, citric acid, tartaric acid, carbonic acid, picric acid, methanesulfonic acid, paratoluenesulfonic acid, and glutamic acid; salts with inorganic bases, such as sodium, potassium, magnesium, calcium, and aluminum; salts with organic bases, such as methylamine, ethylamine, meglumine, and ethanolamine; salts with basic amino acids, such as lysine, arginine, and ornithine; and ammonium salts.

3-(15-Hydroxypentadecyl)-2,4,4-trimethyl-2-cyclohexen-1-one or a salt thereof, which is an active ingredient of the therapeutic agent of the present invention, can be produced in various dosage forms by using one or more pharmaceutically acceptable carriers by a commonly known pharmaceutical method. The dosage form is not particularly limited, and examples include oral agents such as tablets, coated tablets, pills, powders, granules, capsules, liquids, suspensions, and emulsions; and parenteral agents such as injections or suppositories.

The pharmaceutical carrier can be any of various organic or inorganic carrier substances conventionally used as materials for pharmaceutical preparations. Examples include excipients, binders, disintegrants, lubricants, coating agents, and the like used in solid preparations; and solubilizing agents, suspending agents, isotonizing agents, pH adjusters ▪ buffers, soothing agents, and the like used in liquid preparations. Additives for pharmaceutical preparations, such as antiseptics, antioxidants, coloring agents, flavoring ▪ odor-masking agents, and stabilizers, can also be used, if necessary.

Examples of excipients include lactose, sucrose, D-mannitol, starch, crystalline cellulose, calcium silicate, and the like.

Examples of binders include hydroxypropyl cellulose, methyl cellulose, polyvinylpyrrolidone, candy powder, hypromellose, and the like.

Examples of disintegrators include sodium starch glycolate, carmellose calcium, croscarmellose sodium, crospovidone, low-substituted hydroxypropyl cellulose, partially pregelatinized starch, and the like.

Examples of lubricants include talc, magnesium stearate, sucrose fatty acid ester, stearic acid, sodium stearyl fumarate, and the like.

Examples of coating agents include ethyl cellulose, aminoalkyl methacrylate copolymer RS, hypromellose, sucrose, and the like.

Examples of solvents include water, propylene glycol, physiological saline, and the like.

Examples of solubilizing agents include polyethylene glycol, ethanol, $\alpha$-cyclodextrin, macrogol 400, polysorbate 80, and the like.

Examples of suspending agents include carrageenan, crystalline cellulose ▪ carmellose sodium, polyoxyethylene hydrogenated castor oil, and the like.

Examples of isotonizing agents include sodium chloride, glycerin, potassium chloride, and the like.

Examples of pH adjusters ▪ buffers include sodium citrate, hydrochloric acid, lactic acid, phosphoric acid,

sodium dihydrogen phosphate, and the like.

Examples of soothing agents include procaine hydrochloride, lidocaine, and the like.

Examples of antiseptics include ethyl paraoxybenzoate, cresol, benzalkonium chloride, and the like.

Examples of antioxidants include sodium sulfite, ascorbic acid, natural vitamin E, and the like.

Examples of coloring agents include titanium oxide, iron sesquioxide, Food Blue No. 1, copper chlorophyll, and the like.

Examples of flavoring ▪ odor-masking agents include aspartame, saccharin, sucralose, l-menthol, mint flavor, and the like.

Examples of stabilizers include sodium pyrosulfite, sodium edetate, erythorbic acid, magnesium oxide, dibutylhydroxytoluene, and the like.

When oral solid preparations are to be prepared, tablets, coated tablets, granules, powders, capsules, or the like can be produced by ordinary methods using excipients, binders, disintegrants lubricants, coloring agents, flavoring ▪ odor-masking agents, etc.

When injections are to be prepared, subcutaneous, intramuscular, or intravenous injections can be produced by an ordinary method using pH adjusters, buffers, stabilizers, isotonizing agents, topical anesthetics, etc.

The amount of Compound 1 or a salt thereof to be incorporated in each of such dosage unit forms varies depending on, for example, the condition of the patient to whom Compound 1 is to be administered, or the dosage form. In general, in the case of oral agents, the amount of Compound 1 per dosage unit form is preferably 0.005 to 1000 mg, more preferably 1 to 800 mg, and even more preferably 5 to 500 mg. In the case of injections, the amount of Compound 1 or a salt thereof per dosage unit form is preferably about 0.001 to 500 mg, more preferably 0.02 to 400 mg, and even more preferably 1 to 250 mg. In the case of suppositories, the amount of Compound 1 or a salt thereof per dosage unit form is preferably about 0.01 to 1,000 mg, more preferably 1 to 800 mg, and even more preferably 5 to 500 mg. The patient to whom Compound 1 or a salt thereof is to be administered is a human.

The daily dosage of the active ingredient in such a dosage form varies depending on the condition, body weight, age, sex, etc. of the patient, and cannot be generalized. For example, the daily dosage of the active ingredient for a human adult may generally be about 0.005 to 5000 mg, preferably 0.01 to 2000 mg, even more preferably 10 to 1600 mg, still even more preferably 20 to 800 mg, further even more preferably 100 to 400 mg, still further even more preferably 200 to 400 mg, and most preferably 400 mg. This dosage is preferably administered in one dose, or in about two to four divided doses, per day, and more preferably administered in two divided doses per day.

The present invention provides a method using a residual urine volume as an indicator for a therapeutic effect of 3-(15-hydroxypentadecyl)-2,4,4-trimethyl-2-cyclohexen-1-one or a salt thereof on a lower urinary tract disorder, wherein when the residual urine volume is equal to or greater than coefficient T ($40 \leq T \leq 110$) mL, the treatment using 3-(15-hydroxypentadecyl)-2,4,4-trimethyl-2-cyclohexen-1-one or a salt thereof is determined to be effective.

As described above, in the method of the present invention, when a patient has a residual urine volume that is equal to or greater than coefficient T (mL), a treatment using 3-(15-hydroxypentadecyl)-2,4,4-trimethyl-2-cyclohexen-1-one or a salt thereof is determined to be effective. Coefficient T can be set within the range of $40 \leq T \leq 110$. Accordingly, the method of the present invention includes, for example, a method comprising determining a treatment using 3-(15-hydroxypentadecyl)-2,4,4,4-trimethyl-2-cyclohexen-1-one or a salt thereof to be effective when a patient has a residual urine volume that is equal to or greater than a coefficient of 50 (mL). In a preferred embodiment, coefficient T can be set within the range satisfying $70 \leq T \leq 110$, and more preferably the range satisfying $90 \leq T \leq 110$.

The present invention further provides a method using a residual urine volume as an indicator for a therapeutic effect of 3-(15-hydroxypentadecyl)-2,4,4-trimethyl-2-cyclohexen-1-one or a salt thereof on a lower urinary tract disorder, the method comprising determining a treatment using 3-(15-hydroxypentadecyl)-2,4,4-trimethyl-2-cyclohexen-1-one or a salt thereof to be ineffective when a patient has a residual urine volume that is less than coefficient T (mL) described above.

The present invention provides a method using a residual urine volume as an indicator for a therapeutic effect of 3-(15-hydroxypentadecyl)-2,4,4-trimethyl-2-cyclohexen-1-one or a salt thereof on a disease with reduced voiding efficiency, wherein when the residual urine volume is equal to or greater than coefficient T ($40 \leq T \leq 110$) mL, a treatment using 3-(15-hydroxypentadecyl)-2,4,4-trimethyl-2-cyclohexen-1-one or a salt thereof is determined to be effective.

As described above, in the method of the present invention, when a patient has a residual urine volume that is equal to or greater than coefficient T (mL), a treatment using 3-(15-hydroxypentadecyl)-2,4,4,4-trimethyl-2-cyclohexen-1-one or a salt thereof is determined to be effective. Coefficient T can be set within the range satisfying $40 \leq T \leq 110$. Accordingly, the method of the present invention includes, for example, a method of determining a treatment using 3-(15-hydroxypentadecyl)-2,4,4,4-trimethyl-2-cyclohexen-1-one or a salt thereof to be effective when a patient has a residual urine volume that is equal to or greater than a coefficient of 50 (mL). In a preferred embodiment, coefficient T can be set within the range satisfying $70 \leq T \leq 110$, and more preferably the range satisfying $90 \leq T \leq 110$.

The present invention further provides a method using a residual urine volume as an indicator for a therapeutic effect of 3-(15-hydroxypentadecyl)-2,4,4-trimethyl-2-cyclohexen-1-one or a salt thereof on a disease with reduced voiding

efficiency, wherein when a patient has a residual urine volume that is less than coefficient T (mL) described above, a treatment using 3-(15-hydroxypentadecyl)-2,4,4-trimethyl-2-cyclohexen-1-one or a salt thereof is determined to be ineffective.

When pharmacokinetics vary depending on the difference in drug dosage form, formulation additives, etc., the daily dosage can be increased or decreased to make the pharmacokinetics comparable to those exhibited in a given dosage form.

The present invention is described in more detail below with reference to Test Examples. However, the present invention is not limited thereto.

Examples

Patients having detrusor underactivity (DU) and at least one voiding symptom associated with DU, such as abdominal straining to void, slow urinary stream, intermittent stream, residual urine, and reduced voiding efficiency, were used as subjects. Using such subjects, a placebo-controlled double-blind comparative study was performed to examine the effectiveness and safety of Compound 1 (this test was performed using patients also having overactive bladder as subjects). Under double-blind conditions (in which neither the doctor who prescribed the drug nor the patients knew which drug was administered to each patient), 200 mg of Compound 1 or a placebo was orally administered twice a day after a meal for 12 weeks to evaluate the effectiveness and safety of Compound 1.

To evaluate the effectiveness, BVE (bladder voiding efficiency) was evaluated. BVE (%) was calculated by the following formula:

$$\text{Voided volume}/(\text{Voided volume} + \text{Residual urine volume}) \times 100.$$

The voided volume refers to the amount of urine per urination. The residual urine volume refers to the amount of urine that resides in the bladder as measured by transabdominal ultrasonography after completion of urination. The difference between the baseline (before administration) and the measurement value after 12 weeks of the administration was evaluated by using a paired t-test, whereas the difference in amount of BVE variation between the Compound 1 group and the placebo group was evaluated by using Student's t-test. As shown in Table 1, the mean BVE change after 12 weeks of the administration in the Compound 1 group was 10.91, which was greater than that of the placebo group, but was not recognized as significantly difference from that of the placebo group (p = 0.178).

Table 1

Change in BVE (%) in the entire group of patients having lower urinary tract disorders

| | | Compound 1 | Placebo |
|---|---|---|---|
| Baseline | | | |
| | N | 52 | 24 |
| | Mean(S.D.) | 55.43 (26.82) | 63.71 (21.80) |
| Value at Week 12 | | | |
| | N | 48 | 23 |
| | Mean(S.D.) | 66.79 (27.04) | 65.69 (28.98) |
| | P Value (Paired t-test)* | 0.006 | 0.570 |
| Change from Baseline at Week 12 | | | |
| | N | 48 | 23 |
| | Mean(S.D.) | 10.91 (26.48) | 2.42 (20.09) |
| | Mean Difference | 8.49 | |
| | P Value (Student's t-test) | 0.178 | |

Analysis Set: FAS
P Value (Paired t-test)* :
Week 12 : Value at Week 12 vs Baseline
Mean Difference = Compound 1 - Placebo

To study the effect of Compound 1 on voiding effectiveness, BVE changes were evaluated only in a group of patients with more severe lower urinary tract disorder with a residual urine volume of 50 mL or more. As shown in Table 2, a significant increase in BVE after 12 weeks of the administration was observed in the Compound 1 group, as compared

to the BVE before administration (p < 0.001). In contrast, no tendency toward increased BVE was observed in the placebo group (p = 0.489). Further, the mean BVE change after 12 weeks of the administration in the Compound 1 group was 18.41, and a significant difference from that of the placebo group was also observed (p = 0.031).

Table 2

Change in BVE (%) in a group of only patients having more severe lower urinary tract disorders with a residual urine volume of 50 mL or more

| | | Compound 1 | Placebo |
|---|---|---|---|
| Baseline | | | |
| | N | 32 | 15 |
| | Mean(S.D.) | 42.77 (24.66) | 55.95 (23.70) |
| Value at Week 12 | | | |
| | N | 29 | 15 |
| | Mean(S.D.) | 61.66 (30.45) | 58.83 (26.15) |
| | P Value (Paired t-test.)* | <0.001 | 0.489 |
| Change from Baseline at Week 12 | | | |
| | N | 29 | 15 |
| | Mean(S.D.) | 18.41 (24.39) | 2.88 (15.70) |
| | Mean Difference | 15.53 | |
| | P Value (Student's t-test) | 0.031 | |

Analysis Set: FAS
P Value (Paired t-test)* :
Week 12 : Value at Week 12 vs Baseline
Mean Difference = Compound 1 - Placebo

To further study the effect of Compound 1 on voiding efficiency in more detail, BVE changes were evaluated only in a group of patients having more severe lower urinary tract disorders with a residual urine volume of 100 mL or more. As shown in Table 3, a significant increase in BVE after 12 weeks of the administration was observed in the Compound 1 group, as compared to the BVE before administration (p < 0.001). In contrast, no tendency toward increased BVE was observed in the placebo group (p = 0.708). Further, the mean BVE change after 12 weeks of the administration in the Compound 1 group was 23.57, and a significant difference from that of the placebo group (p = 0.025) was observed. Further, the difference in amount of BVE change between the Compound 1 group and the placebo group after 12 weeks of the administration was 21.47, which was greater than the difference therebetween in a group of patients with a residual urine volume of 50 mL or more (15.53; see Table 2).

Table 3

Change in BVE (%) in a group of only patients having even more severe lower urinary tract disorders with a residual urine volume of 100 mL or more

| | | Compound 1 | Placebo |
|---|---|---|---|
| Baseline | | | |
| | N | 22 | 10 |
| | Mean(S.D.) | 32.10 (20.16) | 44.03 (19.42) |
| Value at Week 12 | | | |
| | N | 19 | 10 |
| | Mean(S.D.) | 54.71 (33.78) | 46.13 (18.72) |
| | P Value (Paired t-test)* | <0.001 | 0.708 |
| Change from Baseline at Week 12 | | | |
| | N | 19 | 10 |
| | Mean(S.D.) | 23.57 (25.54) | 2.10 (17.18) |
| | Mean Difference | 21.47 | |

(continued)

Change in BVE (%) in a group of only patients having even more severe lower urinary tract disorders with a residual urine volume of 100 mL or more

| | | Compound 1 | Placebo |
|---|---|---|---|
| | P Value (Student's t-test) | 0.025 | |

Analysis Set: FAS
P Value (Paired t-test)* :
Week 12 : Value at Week 12 vs Baseline
Mean Difference = Compound 1 - Placebo

BVE changes in a group of only patients having lower urinary tract disorders with a residual urine volume of less than 50 mL was evaluated. As shown in Table 4, no significant increase in BVE after 12 weeks of the administration was observed (p = 0.928) .

Table 4

Change in BVE (%) in a group of only patients having lower urinary tract disorders with a residual urine volume of less than 50 mL

| | | Compound 1 | Placebo |
|---|---|---|---|
| Baseline | | | |
| | N | 20 | 9 |
| | Mean(S.D) | 75.68 (15.37) | 76.64 (9.12) |
| Value at Week 12 | | | |
| | N | 19 | 8 |
| | Mean(S.D) | 74.62 (18_94) | 78 54 (31.34) |
| | P Value (Paired t-test)* | 0.928 | 0.880 |
| Change from Baseline at Week 12 | | | |
| | N | 19 | 8 |
| | Mean(S.D.) | -0.55 (25.99) | 1.54 (27.81) |
| | Mean Difference | -2.09 | |
| | P Value (Student's t-test) | 0.853 | |

Analysis Set: FAS
P Value (Paired t-test)* :
Week 12 : Value at Week 12 vs Baseline
Mean Difference = Compound 1 - Placebo

The above results show that while Compound 1 did not significantly increase the voiding efficiency in patients with a residual urine volume of less than 50 mL, Compound 1 significantly increased the voiding efficiency in patients having more severe lower urinary tract disorders with a residual urine volume of 50 mL or more or with a residual volume of 100 mL or more.

To study the safety of Compound 1 in patients having lower urinary tract disorders, adverse events (any unfavorable medical event that occurred in patients who participated in this study; whether or not there is a causal relationship with the active ingredient) and adverse drug reactions (adverse events that are determined to "have a reasonable possibility" of a causal relationship with the drug) were measured. As shown in Table 5, the incidence of adverse events in the Compound 1 group was 46.2%, and no significant difference from that in the placebo group (37.5%) was observed. In the compound 1 group, no adverse effects were exhibited. The above results confirmed that oral administration of 200 mg of Compound 1 twice a day for 12 weeks has no safety problems and this compound is well tolerated.

Table 5

Percentage of incidence of adverse events and side effects

| | | Adverse Events | Adverse Drug Reactions |
|---|---|---|---|
| Group | N | N (%) | N (%) |
| Compound 1 | 52 | 24 (46.2) | 0 (0.0) |

(continued)

| Percentage of incidence of adverse events and side effects | | | |
|---|---|---|---|
| | | Adverse Events | Adverse Drug Reactions |
| Group | N | N (%) | N (%) |
| Placebo | 24 | 9 (37.5) | 0 (0.0) |
| Analysis Set: All Treated Patients (Treatment Period) | | | |

## Claims

1. A therapeutic agent for a voiding symptom in a patient having a lower urinary tract disorder with a residual urine volume of 50 mL or more, the therapeutic agent comprising 3-(15-hydroxypentadecyl)-2,4,4-trimethyl-2-cyclohexen-1-one or a salt thereof.

2. An agent for improving voiding efficiency in a patient having a lower urinary tract disorder with a residual urine volume of 50 mL or more, the agent comprising 3-(15-hydroxypentadecyl)-2,4,4-trimethyl-2-cyclohexen-1-one or a salt thereof.

3. The therapeutic agent according to claim 1, wherein the patient has a lower urinary tract disorder with a residual urine volume of 100 mL or more.

4. The agent for improving voiding efficiency according to claim 2, wherein the patient has a lower urinary tract disorder with a residual urine volume of 100 mL or more.

5. A method using a residual urine volume as an indicator for a therapeutic effect of 3-(15-hydroxypentadecyl)-2,4,4-trimethyl-2-cyclohexen-1-one or a salt thereof on a lower urinary tract disorder, the method comprising determining a treatment using 3-(15-hydroxypentadecyl)-2,4,4-trimethyl-2-cyclohexen-1-one or a salt thereof to be effective when the residual urine volume is equal to or greater than coefficient T ($40 \leq T \leq 110$) mL.

6. A therapeutic agent for treating a voiding symptom in a patient having a disease with reduced voiding efficiency with a residual urine volume of 50 mL or more, the therapeutic agent comprising 3-(15-hydroxypentadecyl)-2,4,4-trimethyl-2-cyclohexen-1-one or a salt thereof.

7. An agent for improving voiding efficiency in a patient having a disease with reduced voiding efficiency with a residual urine volume of 50 mL or more, the therapeutic agent comprising 3-(15-hydroxypentadecyl)-2,4,4-trimethyl-2-cyclohexen-1-one or a salt thereof.

8. The therapeutic agent according to claim 6, wherein the patient has a disease with reduced voiding efficiency with a residual urine volume of 100 mL or more.

9. The agent for improving voiding efficiency according to claim 7, wherein the patient has a disease with reduced voiding efficiency with a residual urine volume of 100 mL or more.

10. A method using a residual urine volume as an indicator for a therapeutic effect of 3-(15-hydroxypentadecyl)-2,4,4-trimethyl-2-cyclohexen-1-one or a salt thereof on a disease with reduced voiding efficiency, the method comprising determining a treatment using 3-(15-hydroxypentadecyl)-2,4,4-trimethyl-2-cyclohexen-1-one or a salt thereof to be effective when the residual urine volume is equal to or greater than coefficient T ($40 \leq T \leq 110$) mL.

11. A method for treating a voiding symptom in a patient having a lower urinary tract disorder with a residual urine volume of 50 mL of more, the method comprising administering an effective amount of 3-(15-hydroxypentadecyl)-2,4,4-trimethyl-2-cyclohexen-1-one or salt thereof to the patient.

12. 3-(15-Hydroxypentadecyl)-2,4,4-trimethyl-2-cyclohexen-1-one or a salt thereof for use in the treatment of a patient having a lower urinary tract disorder with a residual urine volume of 50 mL or more.

**INTERNATIONAL SEARCH REPORT**

International application No.
PCT/JP2021/025056

A. CLASSIFICATION OF SUBJECT MATTER
Int.Cl. A61K31/122(2006.01)i, A61P13/02(2006.01)i, A61P13/10(2006.01)i
FI: A61K31/122, A61P13/02, A61P13/10

According to International Patent Classification (IPC) or to both national classification and IPC

B. FIELDS SEARCHED

Minimum documentation searched (classification system followed by classification symbols)
Int.Cl. A61K31/122, A61P13/02, A61P13/10

Documentation searched other than minimum documentation to the extent that such documents are included in the fields searched

| | |
|---|---|
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)
JSTPlus/JMEDPlus/JST7580(JDreamIII),
CAplus/REGISTRY/MEDLINE/EMBASE/BIOSIS(STN)

C. DOCUMENTS CONSIDERED TO BE RELEVANT

| Category* | Citation of document, with indication, where appropriate, of the relevant passages | Relevant to claim No. |
|---|---|---|
| X<br>Y<br>A | JP 2002-241271 A (MEIJI MILK PROD CO., LTD.) 28 August 2002 (2002-08-28), claim 1, experimental examples 1, 2 | 12<br>10<br>1-9, 11 |
| X<br>Y<br><br>A | WO 2015/046377 A1 (TAIHO PHARMACEUTICAL CO., LTD.) 02 April 2015 (2015-04-02), claim 1, experimental examples 1-3 | 12<br>1, 3, 5-6, 8, 10-11<br>2, 4, 7, 9 |
| X<br>Y<br>A | WO 2017/022787 A1 (TAIHO PHARMACEUTICAL CO., LTD.) 09 February 2017 (2017-02-09), claims 1-12, experimental examples 1, 2 | 12<br>10<br>1-9, 11 |
| Y | 野尻佳克, 国立長寿医療研究センターでのBVI6100の使用, Sysmex Journal Web, 2012, vol. 13, no. 3, pp. 1-8, particularly, p. 1, left column, ll. 4-6, (NOJIRI, Yoshikatsu, Use of BVI6100 at national center for geriatrics and gerontology) | 1, 3, 5-6, 8, 10-11 |

☒ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

Date of the actual completion of the international search
06 August 2021

Date of mailing of the international search report
17 August 2021

Name and mailing address of the ISA/
Japan Patent Office
3-4-3, Kasumigaseki, Chiyoda-ku,
Tokyo 100-8915, Japan

Authorized officer

Telephone No.

Form PCT/ISA/210 (second sheet) (January 2015)

**INTERNATIONAL SEARCH REPORT**

International application No.
PCT/JP2021/025056

C (Continuation). DOCUMENTS CONSIDERED TO BE RELEVANT

| Category* | Citation of document, with indication, where appropriate, of the relevant passages | Relevant to claim No. |
|---|---|---|
| Y | 田中玲子 ほか, 超音波を利用した排尿管理システムのための排尿データ記録装置の開発, 平成 23 年 電気学会全国大会講演論文集, 2011, vol. 3, pp. 28, 29, particularly, p. 28, right column, ll. 14, 15, (TANAKA, Reiko et al., Development of the urinary data recorder for the urination management system using ultrasound, The 2011 Annual Meeting Record I.E.E. Japan) | 1, 3, 5-6, 8, 10-11 |
| Y | WO 2000/27383 A1 (TAIHO PHARMACEUTICAL CO., LTD.) 18 May 2000 (2000-05-18), page 1, lines 9-15 | 1, 3, 5-6, 8, 11 |

Form PCT/ISA/210 (second sheet) (January 2015)

**INTERNATIONAL SEARCH REPORT**
Information on patent family members

International application No.
PCT/JP2021/025056

| | | |
|---|---|---|
| JP 2002-241271 A | 28 August 2002 | US 2004/0132829 A1<br>claim 1, experimental examples 1-3<br>WO 2002/066024 A1<br>EP 1368014 A1 |
| WO 2015/046377 A1 | 02 April 2015 | US 2015/0094380 A1<br>claim 1, experimental examples 1-3<br>EP 2853261 A1<br>CN 105579036 A |
| WO 2017/022787 A1 | 09 February 2017 | US 2017/0035705 A1<br>claims 1-2, experimental examples 1, 2<br>EP 3332779 A1<br>CN 107921011 A<br>KR 10-2018-0038003 A |
| WO 2000/27383 A1 | 18 May 2000 | US 6329428 B1<br>column 1, lines 17-38<br>EP 1055426 A1<br>CN 1287485 A<br>KR 10-2001-0033852 A |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2020115743 A
- JP 2021093454 A
- WO 1999008987 A
- WO 2002066024 A
- WO 2015046377 A
- WO 2017022787 A


### Non-patent literature cited in the description


- *Br. J. Urol.,* 1987, vol. 60, 519-22
- *Urology,* 1997, vol. 50 (6A), 57-67
- *J. Urol.,* 1999, vol. 162, 142-146
- *Approach to Lower Urinary Tract Dysfunction, Tokyo,* 2007, 2-8
- *Neurourol. Urodyn.,* 2014, vol. 33, 54-58
- *LUTS,* 2014, vol. 6, 131-137
- *Int Urol. Nephrol.,* 2014, vol. 46 (1), 23-S27
- *Neurourol. Urodyn.,* 2018, vol. 37 (8), 2928-2931